# FASCICULE DE BREVET EUROPEEN

(11) **EP 0 976 384 B1**
(45) Date de publication et mention de la délivrance du brevet: **25.07.2001**
(21) Numéro de dépôt: 99401435.5
(22) Date de dépôt: 11.06.1999
(51) Int. Cl.: A61K 7/00, A61K 7/02

(54) **Composition cosmétique et/ou dermatologique biphasée, utile notamment pour le démaquillage des yeux**
Kosmetische und/oder dermatologische biphasische Zusammensetzung zum Abschminken der Augen
Cosmetic and/or dermatological biphasic composition for eyes make-up removal

(30) Priorité: 01.07.1998 FR 9808416
(43) Date de publication de la demande: 02.02.2000
(73) Titulaire: L'OREAL, 75008 Paris (FR)
(72) Inventeur: Picard, Elisabeth, 78140 Velizy (FR); Bui-Bertrand, Lien, 91600 Savigny/S/Orge (FR)
(74) Mandataire: Rasson, Catherine

(56) Documents cités:
- EP-A- 0 116 422
- EP-A- 0 370 856
- EP-A- 0 446 094
- EP-A- 0 603 080
- EP-A- 0 827 736
- DE-A- 3 340 350
- FR-A- 2 645 740

## Description

L'invention a pour objet une composition cosmétique et/ou dermatologique constituée d'une phase aqueuse et d'une phase huileuse distinctes, contenant comme conservateur, au moins un bromure d'ammonium et de préférence le bromure de myristyl triméthyl ammonium, et l'utilisation de cette composition pour le nettoyage et/ou le démaquillage de la peau, des muqueuses et/ou des yeux, et notamment pour le démaquillage des yeux sensibles.

Il est connu d'utiliser pour le démaquillage des yeux, des compositions se présentant sous forme de deux phases distinctes qui s'émulsionnent par agitation et se démixent au repos. Une telle composition est par exemple décrite dans le document EP-A-370856.

Ces compositions sous forme biphasée contiennent des conservateurs, notamment des chlorures d'ammonium quaternaire tels que le chlorure de benzalkonium. Or, ces composés entraînent parfois des problèmes de tolérance, notamment pour les sujets ayant les yeux sensibles, et de ce fait on cherche à remplacer ces conservateurs par d'autres qui soient mieux tolérés.

En outre, les conservateurs habituellement utilisés dans les compositions se présentant sous forme d'une seule phase sont souvent peu compatibles avec la forme galénique biphasée. En effet, ces conservateurs traditionnels conduisent à des instabilités physico-chimiques donnant lieu à la formation d'un voile ou d'un précipité dans la phase aqueuse ou bien ils perturbent fortement l'interface eau/huile conduisant à un aspect rédhibitoire de la composition biphasée.

Il subsiste donc le besoin de disposer d'une composition biphasée se conservant bien, sans avoir les inconvénients de l'art antérieur, c'est-à-dire tout en ayant un aspect agréable et une bonne stabilité.

Or, la demanderesse a constaté avec étonnement que l'utilisation d'un au moins un bromure d'ammonium et notamment d'un bromure de alkyltriméthylammonium permettait d'obtenir des compositions biphasées stables ayant de bonnes propriétés de conservation, aussi bien physico-chimiques que microbiennes, tout en permettant un bon démaquillage dans des conditions de confort et de fraîcheur très satisfaisantes.

Rien ne laissait supposer que ce conservateur était susceptible d'être utilisé avec succès dans les compositions biphasées.

Le document EP-A-116422 décrit une composition de nettoyage composée d'une phase aqueuse supérieure contenant un détergent qui peut être le bromure de cétyltriméthylammonium, et une phase aqueuse inférieure contenant de l'hexaméthylphosphate de sodium. Cette composition ne comporte pas de phase huileuse.

La présente invention a donc pour objet une composition cosmétique et/ou dermatologique biphasée constituée d'une phase aqueuse et d'une phase huileuse distinctes, caractérisée en ce qu'elle contient au moins un bromure d'ammonium.

Comme bromure d'ammonium utilisable dans la composition de l'invention, on peut utiliser en particulier les bromures d'alkyl-triméthylammonium, le radical alkyl comportant de 1 à 22 atomes de carbone, et plus particulièrement de 8 à 20 atomes de carbone.

Comme bromure d'ammonium utilisable dans la composition de l'invention, on peut citer par exemple le bromure de dodécyl triméthylammonium, le bromure de myristyl triméthylammonium, le bromure d'hexadécyl triméthylammonium et leurs mélanges. On utilise plus particulièrement le bromure de myristyl triméthylammonium.

On peut par exemple utiliser le bromure de myristyl-triméthylammonium (nom CTFA : Mytrimonium bromide) soit tel quel soit dans un mélange le contenant, et notamment en mélange avec d'autres bromures d'ammonium, par exemple dans le mélange de bromure de dodécyl-triméthylammonium, de bromure de myristyl triméthyl ammonium et de bromure d'hexadécyl-triméthylammonium, vendu sous la dénomination Cetrimide par la société FEF CHEMICALS.

Le bromure d'ammonium est présent dans la composition de l'invention en une quantité en une quantité suffisante pour agir en tant que conservateur dans la composition. Ainsi, ce composé est présent en une quantité allant par exemple de 0,005 à 0,5 % du poids total de la composition et de préférence de 0,01 à 0,1 % du poids total de la composition.

Selon un mode préféré de réalisation de l'invention, la composition contient en outre comme autre conservateur, du chlorure de N-(3-chloroallyl)-hexaminium (nom CTFA : quaternium-15) dont l'association avec un bromure est bien tolérée et apporte une excellente conservation. Le chlorure de N-(3-chloroallyl)-hexaminium peut être présent dans la composition de l'invention en une quantité allant de 0 à 0,5 % du poids total de la composition et de préférence de 0,001 à 0,1 % du poids total de la composition.

La composition selon l'invention comprend au moins une phase aqueuse et une phase huileuse distinctes.

La phase aqueuse de la composition de l'invention peut comprendre de l'eau déminéralisée stérile et/ou une eau florale telle que de l'eau de rose, de l'eau de bleuet, de l'eau de camomille ou de l'eau de tilleul, ou une eau thermale ou minérale naturelle, comme par exemple : l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevar-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Avene.

La phase huileuse de la composition selon l'invention comprend au moins une huile, celle-ci pouvant être choisie parmi les huiles minérales, végétales ou synthétiques ou encore les huiles de silicone, et leurs mélanges.

Parmi les huiles minérales pouvant constituer la phase huileuse, on peut notamment citer l'huile de vaseline et les hydrocarbures aliphatiques supérieurs tels que par exemple l'isohexadécane ou l'isododécane ; parmi les huiles végétales, l'huile de jojoba, ainsi que l'huile de carthame ; parmi les huiles de silicone éventuellement volatiles, les cyclométhicones telles que le cyclopentadiméthylsiloxane, et parmi les huiles de synthèse, les palmitates d'alkyle où le groupe alkyle a de 2 à 10 atomes de carbone, tels que le palmitate d'isopropyle, ou le palmitate d'octyle, et les adipates d'alkyle où le groupe alkyle a de 2 à 10 atomes de carbone, tels que l'adipate de di-éthyl-2-hexyle, ou tout autre ester aliphatique comportant de 12 à 20 atomes de carbone, et leurs mélanges.

Selon une forme de réalisation particulière de l'invention, la phase huileuse contient au moins au moins une huile choisie parmi les palmitates d'alkyle, l'isohexadécane, l'isododécane, les huiles de silicone volatiles (cyclométhicones) et leurs mélanges.

Quand la composition contient un palmitate d'alkyle, ce dernier est de préférence présent en une proportion d'au moins 5 % et mieux en une proportion allant de 8 à 30 % du poids total de la composition.

Quand la composition contient une huile de silicone volatile, la proportion de cette huile va par exemple de 5 à 50 % du poids total de la composition.

En outre, l'isohexadécane ou de l'isododécane ou un mélange de ces deux huiles peuvent être présents dans la composition de l'invention en une proportion allant de 5 à 20 % du poids total de la composition.

La composition biphasée selon l'invention peut être exempte de tensioactif. Toutefois, elle peut comprendre aussi au moins un tensioactif dans l'une ou l'autre des phases.

Le tensioactif est de préférence du type anionique, non-ionique, ou amphotère. Il est généralement du type non-ionique. Il est de préférence présent dans la phase aqueuse. Il est de préférence présent en une proportion allant de 0,01 à 10 % (de matière active) en poids du poids total de la composition, et encore plus préférentiellement de 0,025 à 3 % du poids total de la composition.

Parmi les tensioactifs non-ioniques, ceux particulièrement préférés sont :
- les esters gras de sorbitol polyoxyéthylénés tels que le produit vendu sous la dénomination de TWEEN 20^{R} par la Société ATLAS.
- les alcools gras polyoxyéthylénés tels que le produit vendu sous la dénomination de REMCOPAL 21912 AL^{R} par la Société GERLAND.
- les alkylphénols polyoxyéthylénés tels que le produit vendu sous la dénomination de TRITON X 100^{R} par la Société ROHM-HAAS, et
- les condensats d'oxyde d'éthylène et d'oxyde de propylène (nom CTFA : Poloxamer) tels que ceux vendus sous les dénominations de SYNPERONIC PE^{R} par la Société ICI et en particulier ceux référencés L 31, L 64, F 38, F 88, L 92, P 103, F 108 et F 127.

Parmi les tensioactifs anioniques, on peut notamment citer :
- les alkyléthers sulfates tels que le produit vendu sous la dénomination de TEXAPON ASV^{R} par la Société HENKEL,
- les alkylsulfoacétates tels que produits vendu sous la dénomination de LATHANOL LAL^{R} par la Société STEPAN,
- les sulfosuccinates d'alkyle tels que le produit vendu sous la dénomination de SODIUM DIOCTYL SULFOSUCCINATE^{R} par la Société RHONE POULENC,
- les alkylamido sulfosuccinates tels que le produit vendu sous la dénomination de REWODERM S 1333^{R} par la Société REWO,
- les alkylamido polypeptides tels que le produit vendu sous la dénomination de LAMEPON S^{R} par la Société GRUNAU, et
- les acylsarcosinates tels que le produit vendu sous la dénomination de ORAMIX L 30^{R} par la Société SEPPIC.

Parmi les tensioactifs amphotères, on peut notamment citer :
- les alkylamidopropyl diméthylbétaïnes tels que le produit vendu sous la dénomination de TEGO BETAINE L 7^{R} par la Société GOLDSCHMIDT,
- les alkylamidobétaïnes tels que le produit vendu sous la dénomination de INCRONAM 30^{R} par la Société CRODA,
- les dérivés d'imidazoline tels que le produit vendu sous la dénomination de CHIMEXANE HD^{R} par la Société CHIMEX, et
- les N-alkyl-β-imino-dipropionates tels que le produit vendu sous la dénomination de MONATERIC ISA 35^{R} par la Société MONA.

De préférence, le rapport pondéral entre la phase aqueuse et la phase huileuse va de 30/70 à 60/40.

La composition de l'invention contient de manière appropriée un milieu physiologiquement acceptable, c'est-à-dire compatible avec la peau, les muqueuses et/ou les cheveux.

Outre les composés indiqués ci-dessus, la composition selon l'invention peut contenir des adjuvants cosmétiques conventionnels qui se trouveront dans l'une ou l'autre phase selon leur nature hydrophile ou lipophile, tels que par exemple des parfums, des colorants, des agents adoucissants, un tampon, des humectants et éventuellement un électrolyte tel que le chlorure de sodium pour apporter une isotonicité dans la phase aqueuse ou tout autre composé approprié compatible avec la composition biphasée de l'invention.

Parmi les agents humectants, on peut notamment mentionner la glycérine, l'hexylèneglycol et le polyéthylèneglycol 600, ceux-ci étant présents à une concentration inférieure ou égale à 5% et de préférence allant de 0,05 à 2 % du poids total de la composition.

Parmi les agents adoucissants, on peut en particulier citer l'allantoïne, et certains extraits de plantes.

Les compositions décrites ci-dessus peuvent être conditionnées, de façon connue, dans un flacon à un seul compartiment. L'utilisateur doit alors agiter le flacon avant d'en verser le contenu sur un coton. On peut également prévoir que les deux phases de la composition soient introduites dans deux compartiments indépendants d'un même flacon, un système étant prévu pour leur mélange au moment de la distribution. De tels dispositifs sont décrits par exemple dans les documents EP-A-497256 et FR-A-2697233.

Avantageusement, l'invention concerne des compositions pour le soin et en particulier pour le nettoyage et/ou le démaquillage de la peau, des muqueuses telles que les lèvres, et/ou des yeux. Elle convient particulièrement pour le démaquillage des yeux dont les cils comportent du mascara, notamment pour le démaquillage des yeux sensibles, et pour le démaquillage des compositions de maquillage longue tenue et/ou dite "sans transfert" telles que décrites par exemple dans le document FR-A-2,747,566.

Aussi, l'invention a encore pour l'objet un procédé cosmétique de nettoyage et/ou de démaquillage de la peau, des muqueuses et/ou des yeux, caractérisé en ce que l'on applique sur la peau, les muqueuses et/ou les yeux, une composition telle que définie ci-dessus.

L'invention a encore pour objet un procédé cosmétique de démaquillage des yeux sensibles, caractérisé en ce que l'on applique sur les yeux une composition telle que définie ci-dessus.

On va maintenant donner à titre d'illustration, des exemples de compositions selon l'invention. Les pourcentages indiqués sont des pourcentages en poids.

### Exemple 1 : composition biphasique

- Cyclométhicone 28 %
- Isohexadécane 19 %
- Bromure de myristyl triméthyl ammonium 0,005 %
- Poloxamer 184 (CTFA) 0,04 %
- Tampon phosphate 0,04 %
- Chlorure de sodium 0,5 %
- Quaternium-15 0,03 %
- colorants qs
- Eau déminéralisée qsp 100 %

## Revendications

1. Composition cosmétique et/ou dermatologique biphasée constituée d'une phase aqueuse et d'une phase huileuse distinctes, caractérisée en ce qu'elle contient au moins un bromure d'ammonium.

2. Composition selon la revendication 1, caractérisée en ce que le bromure d'ammonium est un bromure d'alkyl-triméthylammonium.

3. Composition selon la revendication 1 ou 2, caractérisée en ce que le bromure d'ammonium est choisi parmi le bromure de dodécyl-triméthylammonium, le bromure de myristyl-triméthylammonium, le bromure d'hexadécyl-triméthylammonium et leurs mélanges.

4. Composition selon l'une quelconque des revendications précédentes, caractérisée en ce que le bromure d'ammonium est le bromure de myristyl-triméthylammonium.

5. Composition selon la revendication précédente, caractérisée en ce que le bromure de myristyl triméthylammonium est en mélange avec le bromure de dodécyl-triméthylammonium et le bromure d'hexadécyl-triméthylammonium.

6. Composition selon l'une quelconque des revendications précédentes, caractérisée en ce que le bromure d'ammonium est présent en-une quantité allant de 0,005 à 0,5 % du poids total de la composition et de préférence de 0,01 à 0,1 % du poids total de la composition.

7. Composition selon l'une quelconque des revendications précédentes, caractérisée en ce qu'elle contient en outre du chlorure de N-(3-chloroallyl)-hexaminium.

8. Composition selon la revendication précédente, caractérisée en ce que le chlorure de N-(3-chloroallyl)-hexaminium est présent en une quantité allant de 0,001 à 0,1 % du poids total de la composition.

9. Composition selon l'une quelconque des revendications précédentes, caractérisée en ce que la phase huileuse contient au moins une huile choisie parmi les huiles de silicone, les hydrocarbures aliphatiques supérieurs et les huiles de synthèse.

10. Composition selon l'une quelconque des revendications précédentes, caractérisée en ce que la phase huileuse contient au moins une huile choisie parmi les palmitates d'alkyle, l'isohexadécane, l'isododécane, les huiles de silicone volatiles et leurs mélanges.

11. Composition selon l'une quelconque des revendications précédentes, caractérisée en ce qu'elle contient au moins un tensioactif.

12. Composition selon la revendication précédente, caractérisée en ce que le tensioactif est présent en une quantité allant de 0,01 à 10 % du poids total de la composition.

13. Composition selon l'une quelconque des revendications précédentes, caractérisée en ce que le rapport pondéral entre la phase aqueuse et la phase huileuse va de 30/70 à 60/40.

14. Procédé cosmétique de nettoyage et/ou de démaquillage de la peau, des muqueuses et/ou des yeux, caractérisé en ce que l'on applique sur la peau, les muqueuses et/ou les yeux, la composition selon l'une quelconque des revendications précédentes.

15. Procédé cosmétique de démaquillage des yeux sensibles, caractérisé en ce que l'on applique sur les yeux une composition selon l'une quelconque des revendications 1 à 13.

## Patentansprüche

1. Zweiphasige kosmetische und/oder dermatologische Zusammensetzung, die aus einer wäßrigen Phase und einer davon getrennt vorliegenden Ölphase besteht, dadurch gekennzeichnet, daß sie mindestens ein Ammoniumbromid enthält.

2. Zusammensetzung nach Anspruch 1, dadurch gekennzeichnet, daß es sich bei dem Ammoniumbromid um ein Alkyltrimethylammoniumbromid handelt.

3. Zusammensetzung nach Anspruch 1 oder 2, dadurch gekennzeichnet, daß das Ammoniumbromid unter Dodecyltrimethylammoniumbromid, Myristyltrimethylammoniumbromid, Hexadecyltrimethylammoniumbromid und ihren Gemischen ausgewählt ist.

4. Zusammensetzung nach einem der vorhergehenden Ansprüche, dadurch gekennzeichnet, daß es sich bei dem Ammoniumbromid um Myristyltrimethylammoniumbromid handelt.

5. Zusammensetzung nach dem vorhergehenden Anspruch, dadurch gekennzeichnet, daß das Myristyltrimethylammoniumbromid im Gemisch mit Dodecyltrimethylammoniumbromid und Hexadecyltrimethylammoniumbromid vorliegt.

6. Zusammensetzung nach einem der vorhergehenden Ansprüche, dadurch gekennzeichnet, daß das Ammoniumbromid in einer Menge im Bereich von 0,005 bis 0,5 % des Gesamtgewichts der Zusammensetzung und vorzugsweise im Bereich von 0,01 bis 0,1 % des Gesamtgewicht der Zusammensetzung enthalten ist.

7. Zusammensetzung nach einem der vorhergehenden Ansprüche, dadurch gekennzeichnet, daß sie außerdem N-(3-Chlorallyl)-hexaminiumchlorid enthält.

8. Zusammensetzung nach dem vorhergehenden Anspruch, dadurch gekennzeichnet, daß das N-(3-Chlorallyl)-hexaminiumchlorid in einer Menge im Bereich von 0,001 bis 0,1 % des Gesamtgewichts der Zusammensetzung enthalten ist.

9. Zusammensetzung nach einem der vorhergehenden Ansprüche, dadurch gekennzeichnet, daß die Ölphase mindestens ein Öl enthält, das unter Siliconölen, höheren aliphatischen Kohlenwasserstoffen und Syntheseölen ausgewählt ist.

10. Zusammensetzung nach einem der vorhergehenden Ansprüche, dadurch gekennzeichnet, daß die Ölphase mindestens ein Öl enthält, das unter Alkylpalmitaten, Isohexadecan, Isododecan, flüchtigen Siliconölen und ihren Gemischen ausgewählt ist.

11. Zusammensetzung nach einem der vorhergehenden Ansprüche, dadurch gekennzeichnet, daß sie mindestens einen grenzflächenaktiven Stoff enthält.

12. Zusammensetzung nach dem vorhergehenden Anspruch, dadurch gekennzeichnet, daß der grenzflächenaktive Stoff in einer Menge im Bereich von 0,01 bis 10 % des Gesamtgewichts der Zusammensetzung enthalten ist.

13. Zusammensetzung nach einem der vorhergehenden Ansprüche, dadurch gekennzeichnet, daß das Gewichtsverhältnis der wäßrigen Phase zu der Ölphase im Bereich von 30/70 bis 60/40 liegt.

14. Kosmetisches Verfahren zum Reinigen und/oder Abschminken der Haut, der Schleimhäute und/oder der Augen, dadurch gekennzeichnet, daß auf die Haut, die Schleimhäute und/oder die Augen die Zusammensetzung nach einem der vorhergehenden Ansprüche aufgetragen wird.

15. Kosmetisches Verfahren zum Abschminken empfindlicher Augen, dadurch gekennzeichnet, daß auf die Augen eine Zusammensetzung nach einem der Ansprüche 1 bis 13 aufgetragen wird.

## Claims

1. Two-phase cosmetic and/or dermatological composition consisting of an aqueous phase and a separate oily phase, characterized in that it contains at least one ammonium bromide.

2. Composition according to Claim 1, characterized in that the ammonium bromide is an alkyltrimethylammonium bromide.

3. Composition according to Claim 1 or 2, characterized in that the ammonium bromide is chosen from dodecyltrimethylammonium bromide, myristyltrimethylammonium bromide and hexadecyltrimethylammonium bromide, and mixtures thereof.

4. Composition according to any one of the preceding claims, characterized in that the ammonium bromide is myristyltrimethylammonium bromide.

5. Composition according to the preceding claim, characterized in that the myristyltrimethylammonium bromide is in a mixture with dodecyltrimethylammonium bromide and hexadecyltrimethylammonium bromide.

6. Composition according to any one of the preceding claims, characterized in that the ammonium bromide is present in an amount ranging from 0.005 to 0.5% relative to the total weight of the composition, and preferably from 0.01 to 0.1% relative to the total weight of the composition.

7. Composition according to any one of the preceding claims, characterized in that it also contains N-(3-chloroallyl)hexaminium chloride.

8. Composition according to the preceding claim, characterized in that the N-(3-chloroallyl)hexaminium chloride is present in an amount ranging from 0.001 to 0.1% relative to the total weight of the composition.

9. Composition according to any one of the preceding claims, characterized in that the oily phase contains at least one oil chosen from silicone oils, higher aliphatic hydrocarbons and synthetic oils.

10. Composition according to any one of the preceding claims, characterized in that the oily phase contains at least one oil chosen from alkyl palmitates, isohexadecane, isododecane, volatile silicone oils and mixtures thereof.

11. Composition according to any one of the preceding claims, characterized in that it contains at least one surfactant.

12. Composition according to the preceding claim, characterized in that the surfactant is present in an amount ranging from 0.01 to 10% relative to the total weight of the composition.

13. Composition according to any one of the preceding claims, characterized in that the weight ratio between the aqueous phase and the oily phase ranges from 30/70 to 60/40.

14. Cosmetic process for cleansing and/or removing make-up from the skin, mucous -membranes and/or the eyes, characterized in that the composition according to any one of the preceding claims is applied to the skin, mucous membranes and/or the eyes.

15. Cosmetic process for removing make-up from sensitive eyes, characterized in that a composition according to any one of Claims 1 to 13 is applied to the eyes.
